# EUROPÄISCHE PATENTSCHRIFT

(11) **EP 3 512 830 B1**
(45) Veröffentlichungstag und Bekanntmachung des Hinweises auf die Patenterteilung: **12.03.2025**
(21) Anmeldenummer: 17758874.6
(22) Anmeldetag: 31.08.2017
(51) Int. Cl.: C07C 209/82, C07C 209/84, C07C 209/90

(54) **VERFAHREN ZUR HYDRIERUNG EINES GEMISCHES IN GEGENWART EINES FARBLOSEN AMINS**
METHOD FOR THE HYDROGENATION OF A MIXTURE IN THE PRESENCE OF A COLOURLESS AMINE
PROCÉDÉ D'HYDROGÉNATION D'UN MÉLANGE EN PRÉSENCE D'UNE AMINE INCOLORE

(30) Priorität: 14.09.2016 EP 16188851
(43) Veröffentlichungstag der Anmeldung: 24.07.2019
(73) Patentinhaber: BASF SE, 67056 Ludwigshafen (DE)
(72) Erfinder: WEICKGENANNT, Andreas, 67056 Ludwigshafen (DE); TAURO, Silvia, 67056 Ludwigshafen (DE); DUEFERT, Alexander, 67056 Ludwigshafen (DE); LADNAK, Viktor, 67056 Ludwigshafen (DE); THIELE, Kai, 2040 Antwerpen (BE)
(74) Vertreter: BASF IP Association
(86) Internationale Anmeldenummer: PCT/EP2017/071855
(87) Internationale Veröffentlichungsnummer: WO 2018/050441

(56) Entgegenhaltungen:
- EP-A1- 0 816 333
- US-A- 5 889 070

## Beschreibung

Die vorliegende Erfindung betrifft ein Verfahren zur Hydrierung eines Gemischs (G1) in Anwesenheit eines Katalysators, wobei das Gemisch (G1) mindestens ein farbloses Amin und mindestens eine farbgebende Komponente enthält. Durch den Hydrierungsschritt wird die mindestens eine farbgebende Komponente zunächst teilweise, vorzugsweise vollständig hydriert, während das farblose Amin, insbesondere Anilin, nicht oder nur in einem sehr geringen Maß hydriert wird. Die die Verfärbung des für sich genommen prinzipiell farblosen Amins verursachenden farbgebenden Komponenten werden also durch Hydrierung entfernt, wodurch eine Aufreinigung des farblosen Amins bzw. des Gemisches (G1) erzielt wird. Anschließend wird das farblose Amin in mindestens einem weiteren Schritt zu einem Diphenylmethanderivat umgesetzt.

Verfahren zur Aufreinigung von Aminen und zur Entfernung farbgebender Komponenten z. B. in Anilin sind im Stand der Technik beschrieben.

In US 3,222,310 wird ein Verfahren zur Hemmung der Farbbildung in Aminen offenbart. Dabei wird farbloses Anilin mit Alkalimetallborhydriden versetzt und ist auch nach Kontakt mit Luftsauerstoff farbstabil. In dieser Offenbarung ist das eingesetzte Anilin bereits farblos, eine Entfernung farbgebender Komponenten ist nicht beschrieben, ebenso wenig die Verwendung von Katalysatoren.

US 2,019,032 offenbart ein Verfahren für aromatische Amine, beispielhaft ist Anilin genannt, die durch die Zugabe geringer Mengen an Maleinsäureanhydrid vor einer letzten Destillation bei Kontakt mit Luftsauerstoff nur eine kaum wahrnehmbare Farbänderung zeigen. Wasserstoff wird nicht genannt.

In US 2009/0240077 A1 wird ein Verfahren zur Herstellung von Di- und Polyaminen der Diphenylmethanreihe durch Umsetzung von Anilin und Formaldehyd in Gegenwart eines sauren Katalysators beschrieben, bei dem das eingesetzte Anilin in der Summe weniger als 0,5 Gew.-%, bezogen auf das Gewicht des eingesetzten Anilins, an Verbindungen enthält, die wenigstens eine Carbonylgruppe enthalten oder die durch Reaktion dieser wenigstens eine Carbonylgruppe enthaltenden Verbindungen mit Anilin entstehen.

In WO 2010/057909 wird ein Verfahren zur Herstellung eines Isocyanats offenbart, umfassend Hydrieren eines ein Amin enthaltenden Gemischs (Gi) in Anwesenheit eines Kupfer enthaltenden Hydrierkatalysators unter Erhalt eines das Amin enthaltenden Gemischs (Gii) und Umsetzen des Gemisches (Gii) mit Phosgen unter Erhalt eines das Isocyanat enthaltenden Gemischs (Gii). Bei dem im Gemisch (Gi) enthaltenen Amin handelt es sich vorzugsweise um Di- oder Polyamine der Diphenylmethanreihe wie MDI, die beispielsweise durch Umsetzung von Anilin mit Formaldehyd erhalten werden. Weiterhin betrifft WO 2010/057909 das Isocyanat, herstellbar nach diesem Verfahren. Es wird nicht offenbart, dass das Gemisch (Gi) selbst eine farbgebende Substanz enthalten kann. Das Gemisch (Gi) enthält typischerweise mindestens eine Vorstufe zu einer farbgebenden Substanz, das heißt, dass erst in der Phosgenierungsstufe daraus eine farbgebende Substanz resultiert. Als Beispiele werden N-formylierte Diamine und/oder N-formylierte Polyamine der Diphenylmethanreihe oder Verbindungen der Stoffklasse der 3,4-Dihydrochinazoline genannt. Auch werden keine Verunreinigungen aus der Herstellung der Vorstufe des Gemisches (Gi) genannt, also Verunreinigungen, die bei der Herstellung von beispielsweise farblosen Monoaminen wie Anilin anfallen können.

In US 5,889,070 A wird ein Verfahren zur Herstellung eines Polyamingemisches offenbart, welches durch Reaktion von Anilin mit Formaldehyd in Gegenwart eines sauren Katalysators erhalten wird. Das Verfahren umfasst einen Schritt, in dem das Polyamingemisch durch katalytische oder chemische Hydrierung in Gegenwart eines Metallkatalysators reduziert wird. Vor diesem Schritt wird das Polyamingemisch aufgereinigt. Es ist nicht offenbart, dass eine Hydrierung bereits vor der Umsetzung zu einem Polyamin durchgeführt wird oder dass nach einer Hydrierung eine Umsetzung zu einem Diphenylmethanderivat durchgeführt wird.

Die im Stand der Technik beschriebenen Verfahren zeigen, dass es einen Bedarf an Aminen gibt, die farblos oder nahezu farblos sind, insbesondere, um sie als möglichst reinen Ausgangstoff für die weitere industrielle Verwendung zur Verfügung zu stellen. Die im Stand der Technik beschriebenen Verfahren haben beispielsweise den Nachteil, dass die Farbbildung in einem Amin wie Anilin lediglich gehemmt wird oder dass nicht ein farbloses Amin wie Anilin, sondern Folgeprodukte davon im späteren Verlauf von Synthesen, in denen ein Amin wie Anilin verwendet wird, aufgereinigt werden. Demnach besteht ein Bedarf an einem Verfahren zur Aufreinigung von Aminen, das farblose oder nahezu farblose Amine zur Verfügung stellt.

Die Aufgabe der vorliegenden Erfindung ist daher, ein Verfahren zur Aufreinigung von Aminen bereitzustellen, wodurch ein aminhaltiges Gemisch, das farblos oder nahezu farblos ist, erhalten werden kann.

Gelöst wird die Aufgabe durch ein Verfahren zur Hydrierung eines Gemisches (G1), welches mindestens ein farbloses Amin und mindestens eine farbgebende Komponente enthält, wobei die mindestens eine farbgebende Komponente ausgewählt ist aus cycloaliphatischen Carbonylverbindungen, cycloaliphatischen Aminen, cycloaliphatischen Iminen und/oder C6-Ring-Aromaten, dadurch gekennzeichnet, dass das Gemisch (G1) unter Anwesenheit eines Katalysators hydriert wird unter Erhalt eines Gemisches (G2), in welchem die mindestens eine farbgebende Komponente zumindest teilweise oder vollständig hydriert ist, dass das im Gemisch (G2) enthaltene farblose Amin in mindestens einem weiteren Schritt zu einem Diphenylmethanderivat und das Diphenylmethanderivat gegebenenfalls in mindestens einem weiteren Schritt zu einem aromatischen Isocyanat umgesetzt wird, vorzugsweise wird das farblose Amin zunächst zu einem Diphenylmethanderivat und dieses anschließend durch Phosgenierung zu einem aromatischen Isocyanat umgesetzt.

Ein großer Vorteil des erfindungsgemäßen Verfahrens ist es, dass durch das erfindungsgemäße Verfahren eine Aufreinigung des Gemisches (G1) möglich ist. Dies ist insbesondere bei der Weiterverarbeitung des durch die Hydrierung erhaltenen Gemisches (G2) wichtig, da je reiner die Komponenten des Gemisches (G2) sind, umso reiner sind die späteren Produkte, die durch Umsetzung des Gemisches (G2) erhalten werden. Dies trägt außerdem erheblich zur Kostensenkung von längeren Synthesen bei. Daher ist es besonders wertvoll, die Reinigung von Edukten, die ganz am Anfang von längeren Synthesen stehen (wie z. B. Anilin), durchzuführen.

Ein weiterer Vorteil des erfindungsgemäßen Verfahrens ist es, dass eine zeit- und kostenintensive Aufarbeitung nach der Hydrierung vermieden werden kann.

Ein zusätzlicher Vorteil des erfindungsgemäßen Verfahrens ist es, dass der Anteil des Amins am Gemisch während der Aufreinigung nicht oder nur unerheblich geringer wird.

So ist es erfindungsgemäß besonders vorteilhaft, wenn Katalysatoren, die Kupfer und/oder Palladium enthalten, bei der Hydrierung des Gemisches (G1) verwendet werden, wodurch die Farbwerte des Amins verbessert werden und somit ein farbloses oder nahezu farbloses Gemisch (G2), enthaltend das entsprechende Amin, zur Verfügung gestellt wird. Weiterhin ist es vorteilhaft, dass der Anteil des farblosen Amins am Gemisch während der Hydrierung nicht oder nur unerheblich geringer wird.

Ein weiterer Vorteil des erfindungsgemäßen Verfahrens ist es, wenn das aufgereinigte Amin zum Beispiel Anilin ist, dass dieses zum Beispiel zur Synthese der Verbindungen Methylendianilin (MDA) und Methylendiphenyldiisocyanat (MDI) eingesetzt werden kann, die dann ebenfalls eine vorteilhafte Farbe aufweisen, was vor allem für die weitere industrielle Verwendung wichtig ist.

Ein weiterer Vorteil des erfindungsgemäßen Verfahrens ist es, dass durch die Aufreinigung einer für viele Verfahren so grundlegenden Komponente wie einem Amin weitere Reinigungsschritte in Folgeverfahren eingespart werden können und diese Verfahren somit wirtschaftlicher werden.

Daneben ist es auch vorteilhaft, dass das durch die Hydrierung erhaltene Gemisch (G2) eine hohe Langzeit-Farbstabilität aufweist.

Im Rahmen der vorliegenden Erfindung bedeutet der Begriff "Aufreinigung" ein Verfahren, bei welchem der Reinheitsgrad des in Gemisch (G1) vorliegenden Amins verbessert wird, insbesondere durch Hydrierung von im Gemisch enthaltenen farbgebenden Komponenten.

Die Begriffe "farblos" und "farbloses Amin", wie sie im Rahmen dieser Anmeldung verwendet werden, bezeichnen die Farbzahl L*, gemessen nach DIN 5033 (2009), von mindestens einer Verbindung, die mindestens 90, bevorzugt mindestens 95, besonders bevorzugt mindestens 98, beträgt.

Die Begriffe "farbgebend" und "farbgebende Komponente", wie sie im Rahmen dieser Anmeldung verwendet werden, bezeichnen die Farbzahl L*, gemessen nach DIN 5033 (2009), von mindestens einer Verbindung, die kleiner 90, bevorzugt höchstens 80, mehr bevorzugt höchstens 75, beträgt.

Im Folgenden wird das erfindungsgemäße Verfahren zur Hydrierung von Aminen näher definiert.

Ein Gemisch (G1), enthaltend mindestens ein farbloses Amin und mindestens eine farbgebende Komponente, wird in Anwesenheit eines Katalysators hydriert.

Das Gemisch (G1) enthält mindestens ein farbloses Amin. Farblose Amine als solche sind dem Fachmann aus dem Stand der Technik bekannt.

Das mindestens eine farblose Amin kann ein primäres Amin, ein aromatisches Amin und/oder ein Monoamin, bevorzugt ein primäres aromatisches Amin, ein primäres Monoamin und/oder ein aromatisches Monoamin, besonders bevorzugt ein primäres aromatisches Monoamin, ganz besonders bevorzugt Anilin, sein. Insbesondere ist das farblose Amin Anilin.

Das im Gemisch (G1) enthaltene mindestens eine farblose Amin liegt vorzugsweise zu mindestens 80 Gew.-%, besonders bevorzugt zu mindestens 90 Gew.-%, ganz besonders bevorzugt zu mindestens 95 Gew.-%, bezogen auf das Gesamtgewicht des Gemisches (G1), vor.

Der Begriff "Gesamtgewicht des Gemisches (G1)", wie er im Rahmen der vorliegenden Anmeldung verwendet wird, bezeichnet die Summe der Gewichte des mindestens einen farblosen Amins und der mindestens einen farbgebenden Komponente im Gemisch (G1). Eventuell zusätzlich vorhandene Bestandteile (Komponenten) wie Lösungsmittel sind hierbei nicht beinhaltet, sondern werden gegebenenfalls in Relation zum Gesamtgewicht des Gemisches (G1) zusätzlich angegeben.

Farbgebende Komponenten im Zusammenhang mit der vorliegenden Erfindung sind dem Fachmann aus dem Stand der Technik bekannt. Diese kommen beispielsweise prozessbedingt in dem Gemisch (G1) vor oder werden beispielsweise durch den Kontakt des Gemisches (G1) bzw. der darin enthaltenen Komponenten mit oxidierenden Medien, zum Beispiel Luftsauerstoff, gebildet.

Die mindestens eine farbgebende Komponente ist ausgewählt aus cycloaliphatischen Carbonylverbindungen, cycloaliphatischen Aminen, cycloaliphatischen Iminen und/oder C6-Ring-Aromaten.

Unter cycloaliphatischen Carbonylverbindungen, cycloaliphatischen Aminen und cycloaliphatischen Iminen werden im Rahmen der vorliegenden Erfindung sowohl die entsprechenden gesättigten als auch die entsprechenden ungesättigten Verbindungen verstanden. Diese Verbindungen können gegebenenfalls substituiert sein, beispielsweise mit einem oder mehreren Alkyl- oder Hydroxyresten.

Unter dem Begriff "C6-Ring-Aromat" wird im Rahmen dieser Anmeldung sowohl unsubstituiertes als auch substituiertes Benzol verstanden.

Wenn es sich um ein substituiertes Benzol handelt, kann dieses an einer oder an mehreren Stellen substituiert sein. Die Substituenten sind unabhängig voneinander vorzugsweise Alkyl-, Hydroxy-, Halogen-, Amino- und/oder Nitro-Reste.

Die mindestens eine farbgebende Komponente im Gemisch (G1) ist vorzugsweise ausgewählt aus Cyclohexylphenylamin-Derivaten, Cyclohexanon-Derivaten, Cyclohexenon-Derivaten, Cyclohexylamin-Derivaten, N-Methylcyclohexylamin-Derivaten, Toluidin-Derivaten, Nitrobenzol-Derivaten, 2-Aminophenol-Derivaten, 1,2-Phenylendiamin-Derivaten, Diphenylamin-Derivaten, oxidierten N-Methylanilin-Derivaten oder nicht oxidierten N-Methylanilin-Derivaten, besonders bevorzugt aus Cyclohexylphenylamin-Derivaten, Cyclohexylamin-Derivaten, N-Methylcyclohexylamin-Derivaten, oxidierten N-Methylanilin-Derivaten oder nicht oxidierten N-Methylanilin-Derivaten.

Der Begriff "Derivat" umschließt dabei sowohl die jeweiligen Stammverbindungen als auch die davon abgeleiteten Verbindungen, bei denen beispielsweise ein oder mehrere H-Atome durch andere funktionelle Gruppen ersetzt werden. Handelt es sich zum Beispiel um ein Cyclohexanon-Derivat, umfasst der Begriff "Derivat" sowohl das reine Cyclohexanon (Stammverbindung), als auch die davon abgeleiteten Verbindungen wie beispielsweise 3-Methyl-Cyclohexanon.

Die im Gemisch (G1) enthaltene mindestens eine farbgebende Komponente liegt vorzugsweise zu höchstens 5 Gew.-%, besonders bevorzugt zu höchstens 2 Gew.-%, ganz besonders bevorzugt zu höchstens 1 Gew.-%, bezogen auf das Gesamtgewicht des Gemisches (G1), vor.

Gemäß einer Ausführungsform der vorliegenden Erfindung handelt es sich bei dem Gemisch (G1) um Recycle-Anilin, wobei das Recycle-Anilin mindestens eine farbgebende Komponente ausgewählt aus cycloaliphatischen Carbonylverbindungen, cycloaliphatischen Aminen, cycloaliphatischen Iminen und/oder C6-Ring-Aromaten enthält.

Weiterhin kann das Gemisch (G1) zusätzlich zu dem mindestens einen farblosen Amin und der mindestens einen farbgebenden Komponente weitere Bestandteile wie beispielsweise Lösemittel enthalten. Sofern solche Bestandteile (Komponenten) im Gemisch (G1) enthalten sind, werden die entsprechenden Mengenangaben dieser Bestandteile zusätzlich in Relation zum Gesamtgewicht des Gemisches (G1) angegeben. Vorzugsweise sind im Gemisch (G1) keine weiteren Bestandteile enthalten oder in einer Menge von maximal 10 %, bezogen auf das Gesamtgewicht des Gemisches (G1), also der Summe aus farblosen Aminen und farbgebenden Komponenten.

Das Gemisch (G1) wird in Anwesenheit eines Katalysators hydriert unter Erhalt eines Gemisches (G2), in welchem die mindestens eine farbgebende Komponente zumindest teilweise oder vollständig hydriert ist.

Der Ausdruck "zumindest teilweise hydriert", wie er im Rahmen dieser Anmeldung verwendet wird, bezeichnet, dass die mindestens eine farbgebende Komponente zu einem Prozentsatz von mindestens 50 Gew.-%, bevorzugt von mindestens 60 Gew.-%, mehr bevorzugt zu mindestens 70 Gew.-%, bezogen auf das Gesamtgewicht der mindestens einen farbgebenden Komponente in Gemisch (G1), hydriert wurde.

Das Gemisch (G2) enthält nach der Hydrierung einen Großteil des farblosen Amins (aus dem Gemisch (G1)), beispielsweise zu mindestens 90 Gew.-%, bevorzugt zu mindestens 94 Gew.-%, mehr bevorzugt zu mindestens 96 Gew.-%, besonders bevorzugt zu mindestens 99 Gew.-% des mindestens einen farblosen Amins, bezogen auf die im Gemisch (G1) enthaltene Gesamtmenge des farblosen Amins.

Die Farbe eines Gemisches kann durch verschiedene Verfahren bestimmt werden, zum Beispiel mit Hilfe der CIE-Farbskala. Diese wird definiert durch die Werte L*, a* und b*. L* ist ein Maß für die Helligkeit der Probe (0-100, 100 ist am hellsten, also farblos), a* bezeichnet den Grün- (negative Werte) bzw. Rot-Anteil (positive Werte), b* bezeichnet den Blau- (negative Werte) bzw. Gelb-Anteil (positive Werte). Bei a* und b* bezeichnet ein Wert von 0 eine farblose Probe.

Der Begriff "Helligkeit" ist im Rahmen der vorliegenden Erfindung äquivalent zu dem Begriff "Farblosigkeit".

Im erfindungsgemäßen Verfahren sind insbesondere die Werte der Farbzahlen L* und b* von Bedeutung.

Die erfindungsgemäßen Gemische (G1) haben bevorzugt eine Farbzahl L*, gemessen nach DIN 5033 (2009), von mindestens 50.

Das mindestens eine farblose Amin, enthalten in Gemisch (G1), hat bevorzugt eine Farbzahl L*, gemessen nach DIN 5033 (2009), von mindestens 90, bevorzugt mindestens 95, besonders bevorzugt mindestens 98.

Die erfindungsgemäßen Gemische (G2) haben bevorzugt eine Farbzahl L*, gemessen nach DIN 5033 (2009), von mindestens 90, besonders bevorzugt von mindestens 95, ganz besonders bevorzugt von mindestens 98.

Bevorzugt haben die erfindungsgemäßen Gemische (G2) eine Farbzahl b^{*}, gemessen nach DIN 5033 (2009), von höchstens 20, besonders bevorzugt von höchstens 15, ganz besonders bevorzugt von höchstens 10.

Im Verfahren der vorliegenden Erfindung wird das Gemisch (G1) in Anwesenheit eines Katalysators hydriert.

Als Katalysatoren kommen grundsätzlich alle geeigneten, dem Fachmann bekannten, Hydrierkatalysatoren in Frage. Im Zusammenhang mit der vorliegenden Erfindung kommen bevorzugt solche Katalysatoren zum Einsatz, die primäre Amine nicht oder nur geringfügig hydrieren.

Im erfindungsgemäßen Verfahren werden vorzugsweise Katalysatoren, die Kupfer, Palladium, Cobalt, Rhenium und/oder Mangan, besonders bevorzugt Kupfer und/oder Palladium, enthalten, verwendet. Die vorgenannten Elemente wie Kupfer bilden also die katalytisch aktive Komponente des entsprechenden Katalysators, der auch noch weitere Komponenten wie Trägermaterialien aufweisen kann.

Der Katalysator, der im erfindungsgemäßen Verfahrens eingesetzt wird, kann Kupfer in einer Menge im Bereich von 0,1 bis 100 Gew.-%, bevorzugt im Bereich von 20 bis 80 Gew.-%, bezogen auf das Gesamtgewicht des Katalysators und berechnet als Metall, und/oder Palladium in einer Menge im Bereich von 0,1 bis 100 Gew.-%, bevorzugt im Bereich von 0.1 bis 30 Gew.-%, bezogen auf das Gesamtgewicht des Katalysators und berechnet als Metall, enthalten.

Hierbei bedeutet der Ausdruck "berechnet als Metall", dass sich die Angabe auf das elementare Metall bezieht.

Wenn der Katalysator Kupfer enthält, kann das Kupfer im Katalysator als Metall oder als Kupferverbindung oder als Gemisch aus mindestens zwei Kupferverbindungen, oder als Gemisch aus Metall und einer Kupferverbindung oder als Gemisch aus Metall und mindestens zwei Kupferverbindungen vorliegen.

Als Kupferverbindungen kommen vorzugsweise Kupferchromat, Kupferchromit, Kupferoxid, Kupfernitrat, Kupfersulfat, Kupferchlorid, Kupferbromid, Kupferiodid, Kupfercarbonat, Kupferacetylacetat, Kupferalkoxid, Kupferaryloxid oder Kupfercarboxylat, besonders bevorzugt Kupferchromit oder Kupferoxid, in Frage.

Wenn der Katalysator Palladium enthält, kann das Palladium im Katalysator als Metall oder als Palladiumverbindung oder als Gemisch aus mindestens zwei Palladiumverbindungen, oder als Gemisch aus Metall und einer Palladiumverbindung oder als Gemisch aus Metall und mindestens zwei Palladiumverbindungen vorliegen.

Die im erfindungsgemäßen Verfahren eingesetzten Katalysatoren können entweder geträgert oder ungeträgert vorliegen. Das Trägermaterial ist ein gegenüber den Reaktionsbedingungen inertes oder im wesentlichen inertes Trägermaterial und ist vorzugsweise ausgewählt aus der Gruppe bestehend aus Kohlenstoff, Siliciumoxid, Aluminiumoxid, Manganoxid, Ceroxid, Zirkonoxid, Lanthanoxid, Titanoxid und Mischungen aus zwei oder mehr dieser Materialien. Bevorzugt liegt der Katalysator geträgert oder als Vollmetallkatalysator vor. Der Katalysator kann sowohl als Pulver wie auch als Formkörper eingesetzt werden, bevorzugt als Formkörper.

Als Hydriermittel wird im erfindungsgemäßen Verfahren vorzugsweise Wasserstoffgas (H₂), besonders bevorzugt technisch reiner Wasserstoff, verwendet.

Unter "technisch rein" wird ein Wasserstoffgehalt von vorzugsweise mindestens 99 Gew.-%, besonders bevorzugt von mindestens 99,5 Gew.-% verstanden, bezogen auf das Gesamtgewicht des Wasserstoffs.

Des Weiteren kann das Hydriermittel auch ein Gemisch aus Wasserstoff und Inertgas sein, wobei das Inertgas Helium, Argon, Kohlenstoffdioxid, Stickstoff oder Neon sein kann. Ebenso können Ameisensäure oder andere vergleichbare wasserstofffreisetzende Verbindungen verwendet werden.

Das Hydrierungsverfahren kann kontinuierlich oder diskontinuierlich (Batch-Verfahren) durchgeführt werden, bevorzugt wird das erfindungsgemäße Verfahren kontinuierlich durchgeführt.

Bei der diskontinuierlichen Durchführung kann die Hydrierung beispielsweise in einem Rührkessel bzw. einem Rührautoklaven, einem Schlaufenreaktor, einem Strahlschlaufenreaktor, einer Blasensäule oder einem Festbettreaktor mit Umpumpkreislauf realisiert werden, bevorzugt wird die Hydrierung in einem Rührkessel bzw. einem Rührautoklaven realisiert. Bei der diskontinuierlichen Durchführung werden das Gemisch (G1) und der Katalysator vorzugsweise vollständig im Reaktor vorgelegt. Dabei kann der Katalysator als Festbett oder in anderer Form eingebracht sein. In dem erfindungsgemäßen Verfahren ist die bevorzugte Ausführungsform eine Suspension von Katalysator im Gemisch (G1).

Bei der kontinuierlichen Durchführung wird die Hydrierung zum Beispiel in einem kontinuierlich betriebenen Rührkesselreaktor, einem kontinuierlich betriebenen Schlaufenreaktor, einem kontinuierlich betriebenen Strahlschlaufenreaktor, einer kontinuierlich betriebenen Blasensäule, einem kontinuierlich betriebenen Festbettreaktor mit Umpumpkreislauf oder einer Rührkesselkaskade durchgeführt. Bei der kontinuierlichen Durchführung wird das Gemisch (G1) kontinuierlich zugeführt und das erhaltene Produktgemisch (G2), welches das aufgereinigte Amin enthält, abgeführt. Bei der kontinuierlichen Verfahrensweise befindet sich der Katalysator vorzugsweise als Festbett im Reaktor und wird nur bei Bedarf erneuert und/oder regeneriert.

Die Hydrierung des Gemischs (G1) kann bei aus dem Stand der Technik bekannten Temperaturen durchgeführt werden. Vorzugsweise wird die Hydrierung des Gemisches (G1) bei einer Temperatur zwischen 90 °C und 300 °C, besonders bevorzugt zwischen 90 °C und 200 °C, ganz besonders bevorzugt zwischen 90 °C und 150 °C, durchgeführt.

Die Hydrierungsdauer liegt dabei bevorzugt zwischen 1 und 100 Stunden, besonders bevorzugt zwischen 5 und 80 Stunden, ganz besonders bevorzugt zwischen 10 und 50 Stunden.

Die Hydrierung wird vorzugsweise so lange durchgeführt, bis das Gemisch (G2) mit gewünschter Qualität erreicht wird.

Der Begriff "gewünschte Qualität" bedeutet in diesem Zusammenhang, dass das Gemisch (G2) nach dem Hydrierungsschritt eine bessere Farbe bzw. einen höheren L*-Wert und/oder einen möglichst niedrigen b*-Wert (0 ist das Optimum) aufweist als das Gemisch (G1).

Das erhaltene Produktgemisch (G2) kann auf übliche, dem Fachmann bekannte Art und Weise aufgearbeitet werden.

Beispielsweise kann das erhaltene Produktgemisch (G2) durch eine Fest-Flüssig-Trennung oder durch Destillation, bevorzugt durch Destillation, weiter aufgearbeitet werden, um das mindestens eine farblose Amin zu isolieren, den Katalysator abzutrennen und/oder die Hydrierungsprodukte aus dem Gemisch (G2) zu entfernen.

Die Abtrennung des Katalysators aus dem Produktgemisch (G2) kann im Fall eines diskontinuierlichen Verfahrens beispielsweise durch eine Fest-Flüssig-Trennung wie Filtration, Sedimentation oder Zentrifugation durchgeführt werden.

Die Destillation des Gemischs (G2) erfolgt vorzugsweise mithilfe von Verdampfern und/oder Kolonnen. Hierfür eignen sich die dem Fachmann bekannten Kolonnen. Bevorzugt sind Packungskolonnen, Bodenkolonnen mit Siebböden, Kolonnen mit dualflow Böden, Kolonnen mit Glockenböden oder mit Ventilböden ausgestattete Rektifikationskolonnen, Trennwandkolonnen oder Dünnschicht- und Fallfilmverdampfer. Die eingesetzte Kolonne hat bevorzugt eine theoretische Bodenzahl (Nₜₕ) von 1 bis 100, besonders bevorzugt von 1 bis 50. Vorzugsweise wird die Destillation in Abwesenheit von Sauerstoff durchgeführt.

Unter dem Begriff "Abwesenheit von Sauerstoff" wird verstanden, dass der Sauerstoffvolumenanteil kleiner als 1 %, bevorzugt kleiner als 0,01 %, besonders bevorzugt kleiner als 0,01 % und ganz besonders bevorzugt kleiner als 0,001 %, bezogen auf das Gesamtvolumen der Destillationskolonne ist.

Die Destillation wird vorzugsweise bei einer Kopftemperatur einem Kopfdruck von 1 bis 100 mbar durchgeführt. Das Verfahren kann diskontinuierlich oder kontinuierlich durchgeführt werden.

Bei der diskontinuierlichen Führung wird das Gemisch (G2) in einem Reaktor, vorzugsweise unter Luftausschluss, vorgelegt und aufgeheizt. Nach Erreichen des stationären Zustandes können Fraktionen entnommen werden.

Bei der kontinuierlichen Destillation wird die Kolonne so eingestellt, dass ein Rücklaufverhältnis von 1 bis unendlich, bevorzugt von 1 bis 10, resultiert.

Als Rücklaufverhältnis wird das Massenverhältnis von in die Kolonne zurückgeführtem und als Destillat abgezogenem Kondensat am Kopf einer Destillationskolonne definiert.

Die kontinuierliche Prozessführung erfolgt mithilfe einer Kolonne, welche bevorzugt mit einem Verdampfer und einem Kopf-Kondensator ausgestattet ist.

In mindestens einem weiteren Verfahrensschritt wird das im Gemisch (G2) enthaltene farblose Amin zu einem Diphenylmethanderivat umgesetzt und das Diphenylmethanderivat kann gegebenenfalls in mindestens einem weiteren Schritt zu einem aromatischen Isocyanat umgesetzt werden. Vorzugsweise wird das farblose Amin zunächst zu einem Diphenylmethanderivat und dieses anschließend durch Phosgenierung zu einem aromatischen Isocyanat umgesetzt.

Wenn es sich bei dem farblosen Amin beispielsweise um Anilin handelt, wird das Anilin in mindestens einem weiteren Schritt zu Methylendianilin (MDA) umgesetzt.

Das Methylendianilin (MDA) kann dann weiter zu Methylendiphenyldiisocyanat (MDI) umgesetzt werden.

Die Synthese von MDI erfolgt typischerweise in einem zweistufigen Prozess, wobei zuerst Anilin mit Formaldehyd zu MDA umgesetzt wird und das MDA anschließend in einem zweiten Schritt mit Phosgen umgesetzt wird. Die Phosgenierung von MDA ist dem Fachmann bekannt und beispielsweise in H. Ullrich "Chemistry and Technology of Isocyanates", John Wiley, 1996 oder WO 2010/057909 beschrieben.

Gemäß einer Ausführungsform der Erfindung enthält das Gemisch (G2) mindestens 90 Gew.-%, bevorzugt mindestens 94 Gew.-%, mehr bevorzugt mindestens 96 Gew.-%, besonders bevorzugt mindestens 99 Gew.-%, Anilin, wobei das Anilin in mindestens einem weiteren Schritt zu Methylendianilin (MDA) umgesetzt wird, das vorzugsweise anschließend zu Methylendiphenyldiisocyanat (MDI) umgesetzt wird.

In einer weiteren bevorzugten Ausführungsform der vorliegenden Erfindung wird das Gemisch (G1) in Gegenwart eines kupferhaltigen und/oder palladiumhaltigen Katalysators hydriert. Das Gemisch (G1) enthält dabei vorzugsweise mindestens 90 Gew.-%, mehr bevorzugt mindestens 94 Gew.-%, noch mehr bevorzugt mindestens 96 Gew.-%, besonders bevorzugt mindestens 99 Gew.-%, Anilin (bezogen auf das Gesamtgewicht des Gemischs (G1)).

Im Gemisch (G2) ist nach der Hydrierung vorzugsweise mindestens 90 Gew.-% Anilin enthalten. Bevorzugt ist dabei, dass < 2 Gew.-%, noch mehr bevorzugt < 1 Gew.-%, besonders bevorzugt < 0,5 Gew.-%, weniger Anilin im Gemisch (G2) vorliegt im Vergleich zum Gemisch (G1) vor der Hydrierung. Weiterhin ist es in dieser Ausführungsform bevorzugt, dass das im Gemisch (G2) enthaltene Anilin gegebenenfalls aufgereinigt wird und zuerst unter Verwendung von Formaldehyd zu MDA umgesetzt wird, worauf anschließend MDA zu MDI umgesetzt wird.

### Beispiele

Die nachfolgenden Beispiele geben exemplarisch wieder, wie das erfindungsgemäße Verfahren ausgeführt werden kann. Das erfindungsgemäße Verfahren beschränkt sich dabei jedoch nicht auf die in den Beispielen gegebenen Umsetzungen.

### Beispiele 1-7: Variation des Katalysators

### Allgemeine Versuchsdurchführung

Anilin (150 g) wird in einen 300 mL Autoklaven gefüllt. Dazu wird Katalysator (1,5 g) gegeben. Der Autoklav wird mehrfach mit Stickstoff gespült und der Stickstoff dann durch reinen Wasserstoff ersetzt. Nach Aufheizen auf 100 °C wird der Wasserstoffdruck auf 40 bar eingestellt und die Mischung für 12 h bei Druck und Temperatur gerührt. Im Anschluss wird der Autoklav abgekühlt und entspannt und das Anilin entnommen, von dem nun die Farbzahl bestimmt wird.

**Tabelle 1**

| Beispiel | Katalysator | Farbzahl Anilin vor Hydrierung | Gehalt Anilin vor Hydrierung | Farbzahl nach Hydrierung | Gehalt Anilin nach Hydrierung |
|---|---|---|---|---|---|
| | | L* : a* : b* | [GC-Flächen%] | L* : a* : b* | [GC-Flächen%] |
| 1 | 60% CuO, 40% Cr₂O₃ | 67,3 : 23,3: 66,2 | 96,6 | 90,3 : 1,9 : 11,5 | 96,1 |
| 2 | 65% CuO,5% La, 30% Al₂O₃ | 67,3 : 23,3: 66,2 | 96,6 | 91,9 : -1,5 : 8,2 | 96,5 |
| 3 | 50% CuO 50% Al₂O₃ | 67,3 : 23,3: 66,2 | 96,6 | 96,6 : -0,5 : 7,8 | 96,6 |
| 4 | 5% Pd 95% C | 67,3 : 23,3: 66,2 | 96,6 | 98,5 : -0,8 : 5,2 | 94,2 |
| 5 | 65% Co, 25% Cu, 10% Mn | 67,3 : 23,3: 66,2 | 96,6 | 98,2: -3,1 : 12,4 | 96,0 |
| 6 | Raney-Ni | 67,3 : 23,3: 66,2 | 97 | 80:10:30 | 51 |
| 7 | 5% Ru 95% ZrO₂ | 67,3 : 23,3: 66,2 | 97 | 82:-0.5:15 | 84 |

Tabelle 1 zeigt, dass der Einsatz von Cu-haltigen Katalysatoren besonders vorteilhaft ist, da die Farbzahl deutlich verbessert werden kann und der Gehalt an Anilin nahezu unverändert bleibt. Der Einsatz von Ni- bzw. Ru-haltigen Katalysatoren hingegen führt zwar zu einem Verlust an Anilin, die Farbzahl wird aber verbessert. Sowohl in der vorstehenden Tabelle 1 als auch im nachfolgenden Text ergibt die Summe des Katalysatorgewichts immer 100 % (berechnet als Gew.-%). Sofern die jeweiligen Angaben weniger als 100 % ergeben, betreffen die fehlenden Prozentangaben das Trägermaterial.

### Beispiel 8: Variation der Reaktionslaufzeit

### Allgemeine Versuchsdurchführung

Anilin (150 g) wird in einen 300 mL Autoklaven gefüllt. Dazu wird ein 50% CuO 50% Al₂O₃-Katalysator (1,5 g) gegeben. Der Autoklav wird mehrfach mit Stickstoff gespült und der Stickstoff dann durch reinen Wasserstoff ersetzt. Nach Aufheizen auf 100 °C wird der Wasserstoffdruck auf 40 bar eingestellt und die Mischung für die angegebene Zeit bei Druck und Temperatur gerührt. Im Anschluss wird der Autoklav abgekühlt und entspannt und das Anilin entnommen, von dem nun die Farbzahl bestimmt wird.

**Tabelle 2**

| Beispiel | Reaktionszeit | Farbzahl Recycle-Anilin vor Hydrierung | Gehalt Anilin vor Hydrierung | Farbzahl nach Hydrierung | Gehalt Anilin nach Hydrierung |
|---|---|---|---|---|---|
| | [h] | L* : a* : b* | [GC-Flächen%] | L* : a* : b* | [GC-Flächen%] |
| 2 | 12 h | 67,3 : 23,3: 66,2 | 96,6 | 91,9 : -1,5 : 8,2 | 96,5 |
| 8 | 48 h | 67,3 : 23,3: 66,2 | 96,6 | 96,8 : 0,0 : 8,1 | 96,4 |

Die Versuche in Tabelle 2 zeigen, dass die Reaktionsdauer einen großen Einfluss auf die erhaltene Farbzahl L* hat, wobei diese mit steigender Reaktionszeit größer wird und der Anilin-Gehalt gleich bleibt. Jedoch mit zunehmender Reaktionszeit der Anteil an Anilin zurückgeht.

### Beispiele 9-12: Variation der Reaktionstemperatur

### Allgemeine Versuchsdurchführung

Anilin (150 g) wird in einen 300 mL Autoklaven gefüllt. Dazu wird ein 50% CuO 50% Al₂O₃-Katalysator (1,5 g) gegeben. Der Autoklav wird mehrfach mit Stickstoff gespült und der Stickstoff dann durch reinen Wasserstoff ersetzt. Nach Aufheizen auf die angegebene Temperatur wird der Wasserstoffdruck auf 40 bar eingestellt und die Mischung für 12 h bei Druck und Temperatur gerührt. Im Anschluss wird der Autoklav abgekühlt und entspannt und das Anilin entnommen, von dem nun die Farbzahl bestimmt wird.

**Tabelle 3**

| Beispiel | Reaktionstemperatur | Farbzahl Anilin vor Hydrierung | Gehalt Anilin vor Hydrierung | Farbzahl nach Hydrierung | Gehalt Anilin nach Hydrierung |
|---|---|---|---|---|---|
| | [°C] | L* : a* : b* | [GC-Flächen%] | L* : a* : b* | [GC-Flächen%] |
| 2 | 100 | 67,3 : 23,3: 66,2 | 96,6 | 91,9 : -1,5 : 8,2 | 96,5 |
| 9 | 130 | 67,3 : 23,3: 66,2 | 96,6 | 98,2 : 0,1 : 4,9 | 96,6 |
| 10 | 150 | 67,3 : 23,3: 66,2 | 96,6 | 98,4 : -0,3 : 4,9 | 97,1 |
| 11 | 180 | 67,3 : 23,3: 66,2 | 96,6 | 99,5 : -0,5 : 3,0 | 96,7 |
| 12 | 200 | 67,3 : 23,3: 66,2 | 96,6 | 99,2 : -0,4 : 2,5 | 96,7 |

Die Ergebnisse der Tabelle 3 zeigen, dass die Farbzahl durch Erhöhung der Reaktionstemperatur verbessert werden kann. Bei 150 °C (Beispiel 10) ist zudem der Gehalt an Anilin am höchsten.

### Beispiel 13: Destillation zur Verbesserung der Farbzahl

Die Farbzahl der hydrierten Proben kann durch Destillation noch erhöht werden. Dazu wird Anilin aus Beispiel 10 mit Hilfe einer Kurzwegdestillation bei 35 mbar und 72 °C Sumpftemperatur destilliert. Der Hauptlauf hat eine Farbzahl von L* = 100,0, a* = -0,1, b* = 0,3.

### Beispiel 14: Kontinuierliche Hydrierung

34 g Katalysator (65% CuO,5% La, 30% Al₂O₃) werden in einen Rohrreaktor gefüllt und dieser mit Stickstoff inertisiert. Im Anschluss wird der Katalysator durch Überleiten von Wasserstoff aktiviert. Das Anilin wird mit variierender Katalysatorbelastung bei variierender Temperatur bei 20 bar Wasserstoffdruck über den Reaktor gefahren. Vom Austrag werden in regelmäßigen Abständen die Farbzahl und die Zusammensetzung bestimmt. In Tabelle 4 sind einige Punkte dieses Versuchs angegeben.

**Tabelle 4**

| Laufzeit | Temperatur [°C] | Katalysatorbelastung [kg Zulauf/L kat*h] | Anteil Anilin am Gemisch [GC Flächen%] | L* | a* | b* |
|---|---|---|---|---|---|---|
| Zulauf | --- | --- | 95.4 | 92.9 | -5,5 | 40.7 |
| 238 | 100 | 0.30 | 95.5 | 94.1 | -0.1 | 15.5 |
| 309 | 100 | 1.00 | 95.3 | 95.3 | 0.1 | 10.8 |
| 380 | 150 | 0.30 | 95.4 | 98.1 | -0.7 | 5.6 |
| 479 | 125 | 0.30 | 95.4 | 97.1 | -0.4 | 7.9 |
| 551 | 125 | 1.00 | 95.4 | 95.2 | -0.2 | 11.5 |
| 575 | 150 | 1.00 | 95.4 | 96.9 | -0.7 | 9.0 |
| 671 | 170 | 0.65 | 95.1 | 95.3 | -1.1 | 10.8 |
| 695 | 185 | 0.65 | 94.5 | 97.3 | -1.1 | 8.7 |
| 719 | 200 | 0.65 | 94.1 | 97.9 | -0.9 | 6.8 |
| 815 | 200 | 1.00 | 94.9 | 97.1 | -0.5 | 8.5 |
| 860 | 150 | 0.65 | 95.3 | 97.0 | 0.2 | 6.8 |
| 884 | 150 | 0.65 | 95.3 | 96.8 | 0.1 | 7.7 |
| 912 | 150 | 0.65 | 95.4 | 97.4 | 0.2 | 6.0 |
| 1028 | 150 | 0.65 | 95.3 | 96.9 | 0.1 | 7.6 |
| 1148 | 150 | 0.65 | 95.4 | 95.9 | 0.0 | 9.7 |
| 1202 | 150 | 0.65 | 95.4 | 95.7 | -0.1 | 11.5 |

Die Versuche gemäß Tabelle 4 zeigen, dass der eingesetzte Katalysator langzeitig stabil ist.

### Bsp 15: Kontinuierliche Hydrierung

21 g Katalysator (50%CuO 50% Al₂O₃) werden in einen Rohrreaktor gefüllt und dieser mit Stickstoff inertisiert. Im Anschluss wird der Katalysator durch Überleiten von Wasserstoff aktiviert. Das Recycleanilin wird mit variierender Katalysatorbelastung bei variierender Temperatur bei 20 bar Wasserstoffdruck über den Reaktor gefahren. Vom Austrag werden in regelmäßigen Abständen die Farbzahl und die Zusammensetzung bestimmt. In Tabelle 5 sind einige Punkte dieses Versuchs angegeben.

**Tabelle 5**

| Laufzeit | Temperatur [°C] | Katalysatorbelastung [kg Zulauf/L kat*h] | Anteil Anilin am Gemisch [GC Flächen%] | L* | a* | b* |
|---|---|---|---|---|---|---|
| Zulauf | --- | --- | 95.5 | 86.6 | -1.2 | 59.6 |
| 113 | 150 | 0.65 | 95.2 | 88.6 | 1.6 | 29.0 |
| 139 | 170 | 0.65 | 95.1 | 89.9 | 0.6 | 25.8 |
| 161 | 190 | 0.65 | 94.7 | 91.6 | -0.1 | 22.8 |
| 281 | 190 | 0.65 | 95.0 | 84.4 | 2.5 | 25.0 |
| 305 | 200 | 0.65 | 94.8 | 93.5 | -1.3 | 19.7 |
| 401 | 210 | 0.65 | 94.8 | 94.9 | -0.3 | 14.3 |
| 427 | 220 | 0.65 | 94.9 | 94.30 | -0.4 | 16.6 |

Bei gleicher Katalysatorbelastung wird der L*-Wert bei steigender Temperatur größer, der Anteil an Anilin sinkt jedoch.

### Beispiel 16: Synthese von MDI mit farbverbessertem Anilin

Anilin wird nach der folgenden Vorschrift in MDI überführt.

Die Synthese von MDA wird in einem 2 L Doppelmantelreaktor, der mit einem Propellerrührer ausgestattet ist, durchgeführt. Das Anilin aus Beispiel 13 wird mit wässriger Salzsäure (32 Gew.-%) versetzt und auf 90 °C erwärmt. Dann wird eine 36,5 Gew.-%ige wässrige Formaldehydlösung zugegeben, wobei die Reaktionstemperatur auf 90 °C gehalten wird. Nach vollständiger Formaldehydzugabe wird die Reaktionsmischung für 2 h auf 120 °C erwärmt und im Anschluss mit 50 %iger wässriger NaOH-Lösung neutralisiert und anschließend zweimal mit Wasser gewaschen.

Das so erhaltene MDA wird in 1300 mL Monochlorbenzol gegeben und tropfenweise innerhalb von 60 Minuten bei 50 °C zu einer Lösung von Monochlorbenzol und Phosgen (100 g) gegeben. Im Anschluss wird die Reaktionsmischung für 30 Minuten auf 100 °C erwärmt, bis sie klar wird. Das überschüssige Phosgen wird durch Anlegen eines 20 mbar Vakuums bei 75 °C entfernt. Das erhaltene MDI wird zunächst für 60 Minuten bei 100 °C und 50 mbar, dann für 60 Minuten bei 180 °C und 20 mbar dechloriert. Nach Abkühlen auf Raumtemperatur werden die Farb- sowie die NCO-Zahl und der Chlorgehalt bestimmt.

**Tabelle 6**

| Beispiel | Anilin | Farbzahl des MDI L* : a* : b* | NCO-Zahl des MDI |
|---|---|---|---|
| 11 | Anilin | 84,3 : -3,2 : 54,9 | 32,4 |
| 13 | Anilin aus Beispiel 11 | 95,0 : -7,2 : 33,2 | 32,7 |

Die Beispiele in Tabelle 6 zeigen, dass die Farbe des MDI durch die Verwendung hydrierten Anilins deutlich verbessert werden kann. Die NCO-Zahl, ein wichtiges Qualitätskriterium bei MDI, bleibt nahezu unverändert.

## Patentansprüche

1. Verfahren zur Hydrierung eines Gemisches (G1), welches mindestens ein farbloses Amin und mindestens eine farbgebende Komponente enthält, wobei die mindestens eine farbgebende Komponente ausgewählt ist aus cycloaliphatischen Carbonylverbindungen, cycloaliphatischen Aminen, cycloaliphatischen Iminen und/oder C6-Ring-Aromaten, **dadurch gekennzeichnet, dass** das Gemisch (G1) in Anwesenheit eines Katalysators hydriert wird unter Erhalt eines Gemisches (G2), in welchem die mindestens eine farbgebende Komponente zumindest teilweise oder vollständig hydriert ist, dass das im Gemisch (G2) enthaltene farblose Amin in mindestens einem weiteren Schritt zu einem Diphenylmethanderivat und das Diphenylmethanderivat gegebenenfalls in mindestens einem weiteren Schritt zu einem aromatischen Isocyanat umgesetzt wird, vorzugsweise wird das farblose Amin zunächst zu einem Diphenylmethanderivat und dieses anschließend durch Phosgenierung zu einem aromatischen Isocyanat umgesetzt.

2. Verfahren gemäß Anspruch 1, **dadurch gekennzeichnet, dass** das mindestens eine farblose Amin ein primäres Amin ist, ein aromatisches Amin und/oder ein Monoamin ist, bevorzugt ein primäres aromatisches Amin, ein primäres Monoamin und/oder ein aromatisches Monoamin ist, besonders bevorzugt ein primäres aromatisches Monoamin ist, ganz besonders bevorzugt Anilin, ist.

3. Verfahren gemäß Anspruch 1 oder 2, **dadurch gekennzeichnet, dass** das Gemisch (G2) eine Farbzahl L* von mindestens 90, bevorzugt von mindestens 95, besonders bevorzugt von mindestens 98, besitzt und/oder dass das Gemisch (G2) eine Farbzahl b* von höchstens 20, bevorzugt von höchstens 15, besonders bevorzugt von höchstens 10, besitzt.

4. Verfahren gemäß einem der Ansprüche 1 bis 3, **dadurch gekennzeichnet, dass** nach der Hydrierung mindestens 90 Gew.-%, bevorzugt mindestens 94 Gew.-%, mehr bevorzugt mindestens 96 Gew.-%, besonders bevorzugt mindestens 99 Gew.-% des mindestens einen farblosen Amins, bezogen auf die im Gemisch (G1) enthaltene Gesamtmenge des farblosen Amins, in Gemisch (G2) vorhanden sind.

5. Verfahren gemäß einem der Ansprüche 1 bis 4, **dadurch gekennzeichnet, dass** im Gemisch (G1) mindestens ein farbloses Amin zu mindestens 80 Gew.-%, bevorzugt zu mindestens 90 Gew.-%, besonders bevorzugt zu mindestens 95 Gew.-% vorliegt, bezogen auf das Gesamtgewicht des Gemisches (G1).

6. Verfahren gemäß einem der Ansprüche 1 bis 5, **dadurch gekennzeichnet, dass** im Gemisch (G1) die mindestens eine farbgebende Komponente zu höchstens 5 Gew.-%, bevorzugt zu höchstens 2 Gew.-%, besonders bevorzugt zu höchstens 1 Gew.-%, bezogen auf das Gesamtgewicht des Gemisches (G1), vorliegt.

7. Verfahren gemäß einem der Ansprüche 1 bis 6, **dadurch gekennzeichnet, dass** der Katalysator Kupfer, Palladium, Cobalt, Rhenium und/oder Mangan, bevorzugt Kupfer und/oder Palladium, enthält.

8. Verfahren gemäß Anspruch 7, **dadurch gekennzeichnet, dass** der Katalysator
i) Kupfer in einer Menge im Bereich von 0,1 bis 100 Gew.-%, bevorzugt im Bereich von 20 bis 80 Gew.-%, bezogen auf das Gesamtgewicht des Katalysators und berechnet als Metall, enthält,
und/oder
ii) Palladium in einer Menge im Bereich von 0,1 bis 100 Gew.-%, bevorzugt im Bereich von 0,1 bis 30 Gew.-%, bezogen auf das Gesamtgewicht des Katalysators und berechnet als Metall, enthält.

9. Verfahren gemäß Anspruch 7 oder 8, **dadurch gekennzeichnet, dass**
i) wenn der Katalysator Kupfer enthält, das Kupfer als Metall, als Kupferverbindung, als Gemisch aus mindestens zwei Kupferverbindungen, als Gemisch aus Metall und einer Kupferverbindung oder als Gemisch aus Metall und mindestens zwei Kupferverbindungen vorliegt, wobei die Kupferverbindungen vorzugsweise ausgewählt sind aus Kupferchromat, Kupferchromit, Kupferoxid, Kupfernitrat, Kupfersulfat, Kupferchlorid, Kupferbromid, Kupferiodid, Kupfercarbonat, Kupferacetylacetat, Kupferalkoxid, Kupferaryloxid oder Kupfercarboxylat, besonders bevorzugt ausgewählt aus Kupferchromit oder Kupferoxid,
und/oder
ii) wenn der Katalysator Palladium enthält, das Palladium als Metall, als Palladiumverbindung, als Gemisch aus mindestens zwei Palladiumverbindungen, als Gemisch aus Metall und einer Palladiumverbindung oder als Gemisch aus Metall und mindestens zwei Palladiumverbindungen vorliegt.

10. Verfahren gemäß einem der Ansprüche 1 bis 9, **dadurch gekennzeichnet, dass** die mindestens eine farbgebende Komponente ausgewählt ist aus Cyclohexylphenylamin-Derivaten, Cyclohexanon-Derivaten, Cyclohexenon-Derivaten, Cyclohexylamin-Derivaten, N-Methylcyclohexylamin-Derivaten, Toluidin-Derivaten, Nitrobenzol-Derivaten, 2-Aminophenol-Derivaten, 1,2-Phenylendiamin-Derivaten, Diphenylamin-Derivaten, oxidierten N-Methylanilin-Derivaten oder nicht oxidierten N-Methylanilin-Derivaten, besonders bevorzugt ist die mindestens eine farbgebende Komponente ausgewählt aus Cyclohexylphenylamin-Derivaten, Cyclohexylamin-Derivaten, N-Methylcyclohexylamin-Derivaten, oxidierten N-Methylanilin-Derivaten oder nicht oxidierten N-Methylanilin-Derivaten.

11. Verfahren gemäß einem der Ansprüche 1 bis 10, **dadurch gekennzeichnet, dass**
i) die Temperatur während der Hydrierung zwischen 90°C und 300 °C, bevorzugt zwischen 90 °C und 200 °C, besonders bevorzugt zwischen 90 °C und 150 °C, liegt,
und/oder
ii) die Dauer der Hydrierung zwischen 1 und 100 Stunden, bevorzugt zwischen 5 und 80 Stunden, besonders bevorzugt zwischen 10 und 50 Stunden, liegt,
und/oder
iii) die Hydrierung unter Verwendung von Wasserstoffgas (H₂), insbesondere von technisch reinem Wasserstoff, durchgeführt wird, wobei der technisch reine Wasserstoff vorzugsweise einen Wasserstoffgehalt von mindestens 99 Gew.-%, mehr bevorzugt von 99,5 Gew.-%, aufweist, bezogen auf das Gesamtgewicht des Wasserstoffs,
und/oder
iv) das Verfahren kontinuierlich oder diskontinuierlich, bevorzugt kontinuierlich, durchgeführt wird.

12. Verfahren gemäß einem der Ansprüche 1 bis 11, **dadurch gekennzeichnet, dass**
i) der Katalysator geträgert oder ungeträgert, bevorzugt geträgert, vorliegt, wobei das Trägermaterial vorzugsweise ausgewählt ist aus der Gruppe bestehend aus Kohlenstoff, Siliciumoxid, Aluminiumoxid, Manganoxid, Ceroxid, Zirkonoxid, Lanthanoxid, Titanoxid oder Mischungen aus zwei oder mehr dieser Materialien,
und/oder
ii) der Katalysator als Pulver oder als Formkörper, bevorzugt als Formkörper, verwendet wird.

13. Verfahren gemäß einem der Ansprüche 1 bis 12, **dadurch gekennzeichnet, dass** das Gemisch (G2) weiter aufgearbeitet wird, bevorzugt durch Destillation weiter aufgearbeitet wird, um das mindestens eine farblose Amin zu isolieren, den Katalysator abzutrennen, und/oder die Hydrierungsprodukte aus dem Gemisch (G2) zu entfernen.

14. Verfahren gemäß Anspruch 1, **dadurch gekennzeichnet, dass** das Gemisch (G2) mindestens 90 Gew.-%, bevorzugt mindestens 94 Gew.-%, mehr bevorzugt mindestens 96 Gew.-%, besonders bevorzugt mindestens 99 Gew.-%, Anilin enthält und das Anilin in mindestens einem weiteren Schritt zu Methylendianilin (MDA) umgesetzt wird.

15. Verfahren gemäß Anspruch 14, **dadurch gekennzeichnet, dass** Methylendianilin (MDA) zu Methylendiphenyldiisocyanat (MDI) umgesetzt wird.

16. Verfahren gemäß einem der Ansprüche 1 bis 15, **dadurch gekennzeichnet, dass** das Gemisch (G1) Recycle-Anilin ist, wobei das Recycle-Anilin mindestens eine farbgebende Komponente ausgewählt aus cycloaliphatischen Carbonylverbindungen, cycloaliphatischen Aminen, cycloaliphatischen Iminen und/oder C6-Ring-Aromaten enthält.

## Claims

1. A process for hydrogenating a mixture (G1) comprising at least one colorless amine and at least one color-imparting component, where the at least one color-imparting component is selected from among cycloaliphatic carbonyl compounds, cycloaliphatic amines, cycloaliphatic imines and/or C6 ring aromatics, wherein the mixture (G1) is hydrogenated in the presence of a catalyst to give a mixture (G2) in which the at least one color-imparting component is at least partially or completely hydrogenated, the colorless amine present in the mixture (G2) is converted in at least one further step into a diphenylmethane derivative, and optionally, in at least one further step, the diphenylmethane derivative is converted into an aromatic isocyanate, with the colorless amine preferably being firstly converted into a diphenylmethane derivative and this subsequently being converted by phosgenation into an aromatic isocyanate.

2. The process according to claim 1, wherein the at least one colorless amine is a primary amine, an aromatic amine and/or a monoamine, preferably a primary aromatic amine, a primary monoamine and/or an aromatic monoamine, particularly preferably a primary aromatic monoamine, very particularly preferably aniline.

3. The process according to claim 1 or 2, wherein the mixture (G2) has a color number L* of at least 90, preferably at least 95, particularly preferably at least 98, and/or the mixture (G2) has a color number b* of not more than 20, preferably not more than 15, particularly preferably not more than 10.

4. The process according to any of claims 1 to 3, wherein at least 90% by weight, preferably at least 94% by weight, more preferably at least 96% by weight, particularly preferably at least 99% by weight, of the at least one colorless amine, based on the total amount of the colorless amine present in the mixture (G1), is present in the mixture (G2) after the hydrogenation.

5. The process according to any of claims 1 to 4, wherein at least one colorless amine is present in the mixture (G1) in an amount of at least 80% by weight, preferably at least 90% by weight, particularly preferably at least 95% by weight, based on the total weight of the mixture (G1).

6. The process according to any of claims 1 to 5, wherein the at least one color-imparting component is present in the mixture (G1) in an amount of not more than 5% by weight, preferably not more than 2% by weight, particularly preferably not more than 1% by weight, based on the total weight of the mixture (G1).

7. The process according to any of claims 1 to 6, wherein the catalyst comprises copper, palladium, cobalt, rhenium and/or manganese, preferably copper and/or palladium.

8. The process according to claim 7, wherein the catalyst comprises
i) copper in an amount in the range from 0.1 to 100% by weight, preferably in the range from 20 to 80% by weight, based on the total weight of the catalyst and calculated as metal,
and/or
ii) palladium in an amount in the range from 0.1 to 100% by weight, preferably in the range from 0.1 to 30% by weight, based on the total weight of the catalyst and calculated as metal.

9. The process according to claim 7 or 8, wherein
i) if the catalyst comprises copper, the copper is present as metal, as copper compound, as a mixture of at least two copper compounds, as a mixture of metal and a copper compound or as a mixture of metal and at least two copper compounds, where the copper compounds are preferably selected from among copper chromate, copper chromite, copper oxide, copper nitrate, copper sulfate, copper chloride, copper bromide, copper iodide, copper carbonate, copper acetylacetate, copper alkoxide, copper aryloxide and copper carboxylate, particularly preferably selected from among copper chromite and copper oxide,
and/or
ii) if the catalyst comprises palladium, the palladium is present as metal, as palladium compound, as a mixture of at least two palladium compounds, as a mixture of metal and a palladium compound or as a mixture of metal and at least two palladium compounds.

10. The process according to any of claims 1 to 9, wherein the at least one color-imparting component is selected from among cyclohexylphenylamine derivatives, cyclohexanone derivatives, cyclohexenone derivatives, cyclohexylamine derivatives, N-methylcyclohexylamine derivatives, toluidine derivatives, nitrobenzene derivatives, 2-aminophenol derivatives, 1,2-phenylenediamine derivatives, diphenylamine derivatives, oxidized N-methylaniline derivatives or unoxidized N-methylaniline derivatives, with the at least one color-imparting component particularly preferably being selected from among cyclohexylphenylamine derivatives, cyclohexylamine derivatives, N-methylcyclohexylamine derivatives, oxidized N-methylaniline derivatives or unoxidized N-methylaniline derivatives.

11. The process according to any of claims 1 to 10, wherein
i) the temperature during the hydrogenation is in the range from 90°C to 300°C, preferably from 90°C to 200°C, particularly preferably from 90°C to 150°C,
and/or
ii) the duration of the hydrogenation is in the range from 1 to 100 hours, preferably from 5 to 80 hours, particularly preferably from 10 to 50 hours,
and/or
iii) the hydrogenation is carried out using hydrogen gas (H₂), in particular technical-grade hydrogen, where the technical-grade hydrogen preferably has a hydrogen content of at least 99% by weight, more preferably 99.5% by weight, based on the total weight of the hydrogen, and/or
iv) the process is carried out continuously or batchwise, preferably continuously.

12. The process according to any of claims 1 to 11, wherein
i) the catalyst is present in supported or unsupported, preferably supported, form, where the support material is preferably selected from the group consisting of carbon, silicon oxide, aluminum oxide, manganese oxide, cerium oxide, zirconium oxide, lanthanum oxide, titanium oxide and mixtures of two or more of these materials, and/or
ii) the catalyst is used as powder or as shaped bodies, preferably as shaped bodies.

13. The process according to any of claims 1 to 12, wherein the mixture (G2) is worked up further, preferably worked up further by distillation, in order to isolate the at least one colorless amine, to separate off the catalyst and/or to remove the hydrogenation products from the mixture (G2).

14. The process according to claim 1, wherein the mixture (G2) comprises at least 90% by weight, preferably at least 94% by weight, more preferably at least 96% by weight, particularly preferably at least 99% by weight, of aniline and the aniline is converted in at least one further step into methylenedianiline (MDA).

15. The process according to claim 14, wherein methylenedianiline (MDA) is converted into methylene diphenyl diisocyanate (MDI).

16. The process according to any of claims 1 to 15, wherein the mixture (G1) is recycled aniline, where the recycled aniline comprises at least one color-imparting component selected from among cycloaliphatic carbonyl compounds, cycloaliphatic amines, cycloaliphatic imines and/or C6-ring aromatics.

## Revendications

1. Procédé d'hydrogénation d'un mélange (G1), qui contient au moins une amine incolore et au moins un composant colorant, ledit au moins un composant colorant étant choisi parmi les composés carbonyle cycloaliphatiques, les amines cycloaliphatiques, les imines cycloaliphatiques et/ou les aromatiques cycliques en C6, **caractérisé en ce que** le mélange (G1) est hydrogéné en présence d'un catalyseur avec obtention d'un mélange (G2), dans lequel ledit au moins un composant colorant est au moins partiellement ou totalement hydrogéné, **en ce que** l'amine incolore contenue dans le mélange (G2) est transformée dans au moins une autre étape en un dérivé de diphénylméthane et le dérivé de diphénylméthane est le cas échéant transformé dans au moins une autre étape en un isocyanate aromatique, de préférence l'amine incolore est d'abord transformée en un dérivé de diphénylméthane et celui-ci est ensuite transformé par phosgénation en un isocyanate aromatique.

2. Procédé selon la revendication 1, **caractérisé en ce que** ladite au moins une amine incolore est une amine primaire, une amine aromatique et/ou une monoamine, de préférence une amine primaire aromatique, une monoamine primaire et/ou une monoamine aromatique, de manière particulièrement préférée une monoamine primaire aromatique, de manière tout particulièrement préférée l'aniline.

3. Procédé selon la revendication 1 ou 2, **caractérisé en ce que** le mélange (G2) possède un indice de couleur L* d'au moins 90, de préférence d'au moins 95, de manière particulièrement préférée d'au moins 98 et/ou **en ce que** le mélange (G2) possède un indice de couleur b* d'au plus 20, de préférence d'au plus 15, de manière particulièrement préférée d'au plus 10.

4. Procédé selon l'une des revendications 1 à 3, **caractérisé en ce qu'**après l'hydrogénation, au moins 90% en poids, de préférence au moins 94% en poids, plus préférablement au moins 96% en poids, de manière particulièrement préférée au moins 99% en poids de ladite au moins une amine incolore, par rapport à la quantité totale d'amine incolore contenue dans le mélange (G1), sont présents dans le mélange (G2).

5. Procédé selon l'une des revendications 1 à 4, **caractérisé en ce que**, dans le mélange (G1), au moins une amine incolore est présente à raison d'au moins 80% en poids, de préférence à raison d'au moins 90% en poids, de manière particulièrement préférée à raison d'au moins 95% en poids, par rapport au poids total du mélange (G1).

6. Procédé selon l'une des revendications 1 à 5, **caractérisé en ce que**, dans le mélange (G1), ledit au moins un composant colorant est présent à raison d'au plus 5% en poids, de préférence à raison d'au plus 2% en poids, de manière particulièrement préférée à raison d'au plus 1% en poids, par rapport au poids total du mélange (G1).

7. Procédé selon l'une des revendications 1 à 6, **caractérisé en ce que** le catalyseur contient du cuivre, du palladium, du cobalt, du rhénium et/ou du manganèse, de préférence du cuivre et/ou du palladium.

8. Procédé selon la revendication 7, **caractérisé en ce que** le catalyseur contient
i) du cuivre en une quantité dans la plage de 0,1 à 100% en poids, de préférence dans la plage de 20 à 80% en poids, par rapport au poids total du catalyseur et calculé sous forme de métal,
et/ou
ii) du palladium en une quantité dans la plage de 0,1 à 100% en poids, de préférence dans la plage de 0,1 à 30% en poids, par rapport au poids total du catalyseur et calculé sous forme de métal.

9. Procédé selon la revendication 7 ou 8, **caractérisé en ce que**
i) lorsque le catalyseur contient du cuivre, le cuivre se trouve sous forme de métal, sous forme de composé de cuivre, sous forme de mélange d'au moins deux composés de cuivre, sous forme de mélange de métal et d'un composé de cuivre ou sous forme de mélange de métal et d'au moins deux composés de cuivre, les composés de cuivre étant de préférence choisis parmi le chromate de cuivre, le chromite de cuivre, l'oxyde de cuivre, le nitrate de cuivre, le sulfate de cuivre, le chlorure de cuivre, le bromure de cuivre, l'iodure de cuivre, le carbonate de cuivre, l'acétylacétate de cuivre, un alcoxyde de cuivre, un aryloxyde de cuivre ou un carboxylate de cuivre, de manière particulièrement préférée choisis parmi le chromite de cuivre ou l'oxyde de cuivre,
et/ou
ii) lorsque le catalyseur contient du palladium, le palladium se trouve sous forme de métal, sous forme de composé de palladium, sous forme de mélange de deux composés de palladium, sous forme de mélange de métal et d'un composé de palladium ou sous forme de mélange de métal et d'au moins deux composés de palladium.

10. Procédé selon l'une des revendications 1 à 9, **caractérisé en ce que** ledit au moins un composant colorant est choisi parmi les dérivés de cyclohexylphénylamine, les dérivés de cyclohexanone, les dérivés de cyclohexénone, les dérivés de cyclohexylamine, les dérivés de N-méthylcyclohexylamine, les dérivés de toluidine, les dérivés de nitrobenzène, les dérivés de 2-aminophénol, les dérivés de 1,2-phénylènediamine, les dérivés de diphénylamine, les dérivés oxydés de N-méthylaniline ou les dérivés non oxydés de N-méthylaniline, de manière particulièrement préférée, ledit au moins un composant colorant est choisi parmi les dérivés de cyclohexylphénylamine, les dérivés de cyclohexylamine, les dérivés de N-méthylcyclohexylamine, les dérivés oxydés de N-méthylaniline ou les dérivés non oxydés de N-méthylaniline.

11. Procédé selon l'une des revendications 1 à 10, **caractérisé en ce que**
i) la température pendant l'hydrogénation est située entre 90°C et 300°C, de préférence entre 90°C et 200°C, de manière particulièrement préférée entre 90°C et 150°C, et/ou
ii) la durée de l'hydrogénation est située entre 1 et 100 heures, de préférence entre 5 et 80 heures, de manière particulièrement préférée entre 10 et 50 heures,
et/ou
iii) l'hydrogénation est réalisée à l'aide d'hydrogène gazeux (H₂), en particulier d'hydrogène techniquement pur, l'hydrogène techniquement pur présentant de préférence une teneur en hydrogène d'au moins 99% en poids, plus préférablement de 99,5% en poids, par rapport au poids total d'hydrogène,
et/ou
iv) le procédé est réalisé de manière continue ou discontinue, de préférence de manière continue.

12. Procédé selon l'une des revendications 1 à 11, **caractérisé en ce que**
i) le catalyseur se trouve sous forme supportée ou non supportée, le matériau support étant de préférence choisi dans le groupe constitué par le carbone, l'oxyde de silicium, l'oxyde d'aluminium, l'oxyde de manganèse, l'oxyde de cérium, l'oxyde de zirconium, l'oxyde de lanthane, l'oxyde de titane ou les mélanges de deux ou plus de ces matériaux,
et/ou
ii) le catalyseur est utilisé sous forme de poudre ou de corps façonné, de préférence sous forme de corps façonné.

13. Procédé selon l'une des revendications 1 à 12, **caractérisé en ce que** le mélange (G2) est traité davantage, de préférence traité davantage par distillation, ainsi d'isoler ladite au moins une amine incolore, de séparer le catalyseur et/ou d'éliminer les produits d'hydrogénation du mélange (G2).

14. Procédé selon la revendication 1, **caractérisé en ce que** le mélange (G2) contient au moins 90% en poids, de préférence au moins 94% en poids, plus préférablement au moins 96% en poids, de manière particulièrement préférée au moins 99% en poids d'aniline et l'aniline est transformée dans au moins une autre étape en méthylènedianiline (MDA).

15. Procédé selon la revendication 14, **caractérisé en ce que** la méthylènedianiline (MDA) est transformée en diisocyanate de méthylènediphényle (MDI).

16. Procédé selon l'une des revendications 1 à 15, **caractérisé en ce que** le mélange (G1) est de l'aniline recyclée, l'aniline recyclée contenant au moins un composant colorant choisi parmi les composés carbonyle cycloaliphatiques, les amines cycloaliphatiques, les imines cycloaliphatiques, et/ou les aromatiques cycliques en C6.
